# EUROPEAN PATENT SPECIFICATION

(11) **EP 4 497 384 B1**
(45) Date of publication and mention of the grant of the patent: **20.08.2025**
(21) Application number: 24179854.5
(22) Date of filing: 04.06.2024
(51) Int. Cl.: A61B 5/256, A61B 5/282, A61B 5/332, A61B 5/00, A61B 5/0245

(54) **CONNECTOR FOR BIOLOGICAL DATA ACQUISITION**
VERBINDER ZUR ERFASSUNG BIOLOGISCHER DATEN
CONNECTEUR POUR L'ACQUISITION DE DONNÉES BIOLOGIQUES

(30) Priority: 28.07.2023 JP 2023123465
(43) Date of publication of application: 29.01.2025
(73) Proprietor: Japan Aviation Electronics Industry, Ltd., Tokyo 150-0043 (JP)
(72) Inventor: KOMOTO, Tetsuya, Tokyo 150-0043 (JP); MATSUNAGA, Akihiro, Tokyo 150-0043 (JP)
(74) Representative: Qip Patent & Recht Dr. Kuehn & Partner mbB

(56) References cited:
- CN-A- 110 236 494
- US-A1- 2007 093 705
- US-A1- 2012 089 037
- US-A1- 2019 223 801
- US-A1- 2021 128 042
- US-A1- 2023 181 080

## Description

### BACKGROUND OF THE INVENTION

The present invention relates to a connector for biological data acquisition, particularly to a connector for biological data acquisition that is mounted on a mounting object constituted of a wearing article worn by a user and is fitted to a counter connector.

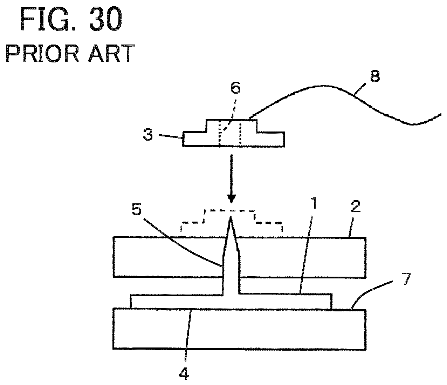

An electrode unit for acquiring a heart rate and other biological data of a user is disclosed in, for example, JP 2020-18783 A. The electrode unit includes a badge portion 1 of pin badge type and a fastener portion 3 used to fasten the badge portion 1 to a garment 2, as shown in FIG. 30. The badge portion 1 includes an electrode portion 4 and a pin 5 formed to protrude on the electrode portion 4. The badge portion 1 is fastened to the garment 2 by sticking the pin 5 into the garment 2 and inserting the tip of the pin 5 protruding from the garment 2 into an insertion hole 6 of the fastener portion 3.

The user's biological data is acquired when the electrode portion 4 is brought into contact with or brought closer to a body surface 7 of the user, and the acquired biological data is transmitted to an external device (not shown) such as a measurement device or a communication device through a cable 8 connected to the fastener portion 3.

However, since biological data is transmitted to the external device through the cable 8, a resistance value in the transmission path increases due to the use of the cable 8, and this may result in lower accuracy of the transmitted biological data.

While it is possible to connect a connector which is not shown to the cable 8 to transmit biological data to a measurement device, a communication device, or the like via the connector, even in this case, an increase in a resistance value due to the use of the cable 8 is not avoidable, and this also causes another problem in that connecting the connector to the electrode unit shown in FIG. 30 results in an increased size of a device to be mounted on the garment 2 to acquire biological data.

US2023/0181080 A1 discloses a bioelectrode wearable for long time. The bioelectrode includes an electrode portion that requires an electric signal of the living body or outputs an electric signal to the living body. The electrode portion is provided with an electrolyte layer that is in close contact with the living body, and is also provided with a sheet-like cover member that covers at least a part or all of the electrolyte layer. The cover member is provided with an opening that penetrates in a thickness direction, thereby discharging moisture accumulated in the electrolyte layer and preventing the swelling and deterioration.

CN 110236494 A discloses a sign information detection processor and physical information detection clothing. US 2021/0128042 A1 discloses a system for capturing biosignals. US 2012/0089037 A1 discloses an ambulatory electrocardiographic monitor with a jumpered sensing electrode and a method of use. US 2007/0093705 A1 discloses an electrode for a living body and a device for detecting living signals.
US 2019/0223801 A1 discloses a patient worn sensor assembly.

### SUMMARY OF THE INVENTION

The present invention has been made to solve the conventional problems described above and aims at providing a small-sized connector for biological data acquisition that is capable of acquiring and transmitting biological data of a user with high accuracy.

A connector for biological data acquisition according to the present invention is defined in claim 1.

### BRIEF DESCRIPTION OF THE DRAWINGS

FIG. 1 is a perspective view of a connector for biological data acquisition according to Embodiment 1 when viewed from an obliquely upper position.
FIG. 2 is a perspective view of the connector for biological data acquisition according to Embodiment 1 when viewed from an obliquely lower position.
FIG. 3 is an assembly view of the connector for biological data acquisition according to Embodiment 1 when viewed from an obliquely upper position.
FIG. 4 is an assembly view of the connector for biological data acquisition according to Embodiment 1 when viewed from an obliquely lower position.
FIG. 5 is a perspective view of a top insulator used in Embodiment 1 when viewed from an obliquely upper position.
FIG. 6 is a perspective view of the top insulator used in Embodiment 1 when viewed from an obliquely lower position.
FIG. 7 is a perspective view of a bottom insulator used in Embodiment 1 when viewed from an obliquely upper position.
FIG. 8 is a perspective view of the bottom insulator used in Embodiment 1 when viewed from an obliquely lower position.
FIG. 9 is a perspective view showing a conductive member used in Embodiment 1.
FIG. 10 is a cross-sectional view showing the conductive member used in Embodiment 1.
FIG. 11 is a perspective view showing a reinforcement sheet used in Embodiment 1.
FIG. 12 is a perspective view showing a mounting object used in Embodiment 1.
FIG. 13 is a perspective cross-sectional view showing the connector for biological data acquisition according to Embodiment 1.
FIG. 14 is a partial, cross-sectional front view showing the connector for biological data acquisition according to Embodiment 1.
FIG. 15 is a perspective view of a connector for biological data acquisition according to Embodiment 2 when viewed from an obliquely upper position.
FIG. 16 is a perspective view of the connector for biological data acquisition according to Embodiment 2 when viewed from an obliquely lower position.
FIG. 17 is an assembly view of the connector for biological data acquisition according to Embodiment 2 when viewed from an obliquely upper position.
FIG. 18 is an assembly view of the connector for biological data acquisition according to Embodiment 2 when viewed from an obliquely lower position.
FIG. 19 is a perspective view showing a contact member used in Embodiment 2.
FIG. 20 is a cross-sectional view showing the contact member used in Embodiment 2.
FIG. 21 is a perspective view showing an electrode member used in Embodiment 2.
FIG. 22 is a cross-sectional view showing the electrode member used in Embodiment 2.
FIG. 23 is a cross-sectional view showing a conductive member used in Embodiment 2.
FIG. 24 is a partial, cross-sectional front view showing the connector for biological data acquisition according to Embodiment 2.
FIG. 25 is a perspective cross-sectional view showing a connector for biological data acquisition according to Embodiment 3.
FIG. 26 is a partial, cross-sectional front view showing a connector for biological data acquisition according to Embodiment 4.
FIG. 27 is a perspective view showing a connector for biological data acquisition according to Embodiment 5 being aligned with a mounting object.
FIG. 28 is a cross-sectional view showing the connector for biological data acquisition according to Embodiment 5 being aligned with the mounting object.
FIG. 29 is a cross-sectional front view showing the connector for biological data acquisition according to Embodiment 5 that is mounted on the mounting object.
FIG. 30 is a cross-sectional view showing a conventional electrode unit.

### DETAILED DESCRIPTION OF THE INVENTION

Embodiments of the present invention are described below based on the accompanying drawings.

### Embodiment 1

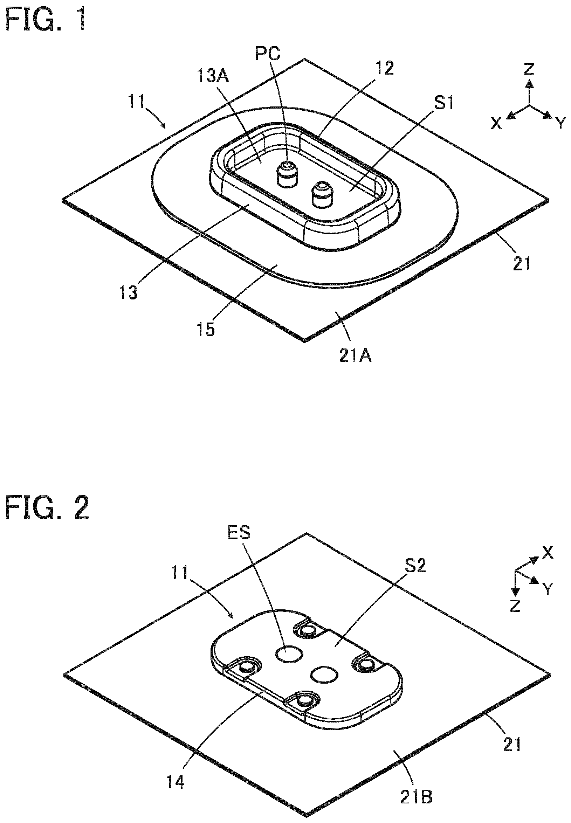

FIGS. 1 and 2 show a connector 11 for biological data acquisition according to Embodiment 1. The connector 11 for biological data acquisition is used as, for example, a garment-side connector for fitting a wearable device and is mounted on cloth 21 of a garment that serves as a mounting object.

The connector 11 for biological data acquisition includes a housing 12 made of an insulating material. In the housing 12, two plug contacts PC are retained to protrude perpendicularly to the cloth 21.

The housing 12 includes a top insulator 13 disposed on a top surface 21A of the cloth 21 and a bottom insulator 14 disposed on a rear surface 21B of the cloth 21. The top surface 21A of the cloth 21 is a surface facing the outside of the garment and faces the opposite side from the body surface of a user when the user wears the garment, and the rear surface 21B of the cloth 21 is a surface facing the inside of the garment and faces the body surface of the user when the user wears the garment.

As shown in FIG. 1, the top insulator 13 is disposed on the top surface 21A of the cloth 21 via a reinforcement sheet 15 and has a recessed portion 13A opening in the opposite direction from the cloth 21. The bottom surface of the recessed portion 13A forms a first exposed surface S1 of flat shape that is exposed on the top surface 21A side of the cloth 21, and the two plug contacts PC protrude perpendicularly to the first exposed surface S1.

As shown in FIG. 2, the bottom insulator 14 is disposed directly on the rear surface 21B of the cloth 21 and has a second exposed surface S2 of flat shape that is exposed on the rear surface 21B side of the cloth 21. Two electrode surfaces ES are disposed and exposed at the second exposed surface S2. The two electrode surfaces ES are arranged to correspond to the two plug contacts PC.

For convenience, the first exposed surface S1 and the second exposed surface S2 are defined as extending along an XY plane, the arrangement direction of the two plug contacts PC is referred to as a Y direction, and the direction in which the two plug contacts PC protrude is referred to as a +Z direction. The first exposed surface S1 of the top insulator 13 is exposed in the +Z direction, and the second exposed surface S2 of the bottom insulator 14 is exposed in the -Z direction. The Z direction coincides with a fitting direction in which the connector 11 for biological data acquisition is fitted to a counter connector (not shown).

The two electrode surfaces ES are exposed in the state of protruding in the -Z direction from the second exposed surface S2 of the bottom insulator 14.

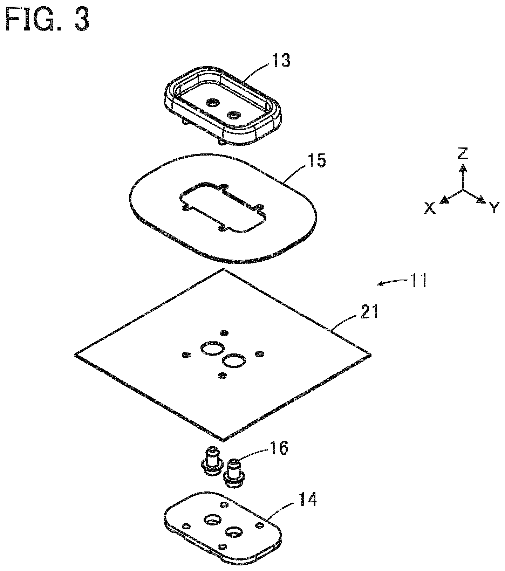

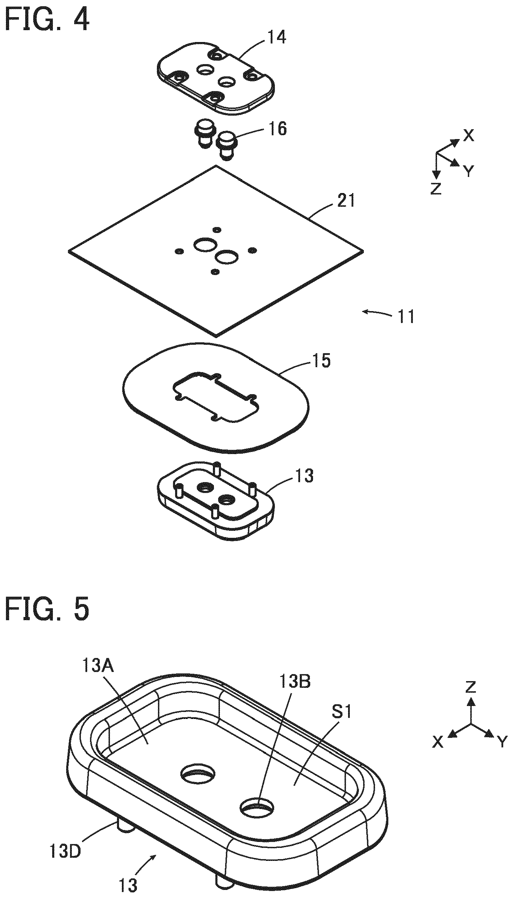

FIGS. 3 and 4 show assembly views of the connector 11 for biological data acquisition. The reinforcement sheet 15 is disposed on the -Z direction side of the top insulator 13, and the cloth 21 is disposed on the -Z direction side of the reinforcement sheet 15. Two conductive members 16 are disposed on the -Z direction side of the cloth 21, and the bottom insulator 14 is disposed on the -Z direction side of the two conductive members 16.

Each conductive member 16 forms both the plug contact PC protruding in the +Z direction from the first exposed surface S1 and the electrode surface ES exposed in the -Z direction from the second exposed surface S2.

As shown in FIG. 5, two contact through-holes 13B penetrating the top insulator 13 in the Z direction are formed within the recessed portion 13A of the top insulator 13. The recessed portion 13A constitutes a counter connector accommodating portion in which part of a counter connector (not shown) is to be accommodated, and the two contact through-holes 13B correspond to the two plug contacts PC.

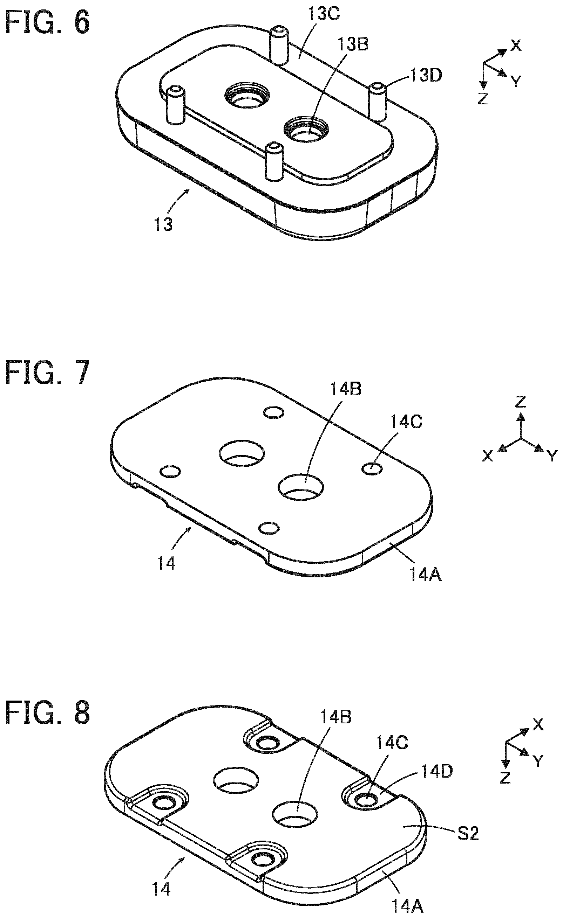

As shown in FIG. 6, a step portion 13C recessed is formed along a peripheral portion of the surface, facing in the -Z direction, of the top insulator 13 to surround the two contact through-holes 13B. The step portion 13C has a depth substantially the same as the thickness dimension of the reinforcement sheet 15. The step portion 13C is provided with four bosses 13D protruding in the -Z direction.

As shown in FIG. 7, the bottom insulator 14 includes a flat plate portion 14A, and the flat plate portion 14A is provided with two electrode through-holes 14B penetrating the flat plate portion 14A in the Z direction. The two electrode through-holes 14B are arranged to correspond to the two contact through-holes 13B of the top insulator 13.

Further, the flat plate portion 14A is provided with four boss through-holes 14C corresponding to the four bosses 13D of the top insulator 13 and penetrating the flat plate portion 14A in the Z direction.

As shown in FIG. 8, recessed portions 14D are formed at the surface, which forms the second exposed surface S2 and faces in the -Z direction, of the bottom insulator 14 to surround the corresponding boss through-holes 14C. The recessed portions 14D are used to accommodate the tips of the bosses 13D of the top insulator 13 that have been passed through the boss through-holes 14C and thermally deformed when the connector 11 for biological data acquisition is assembled.

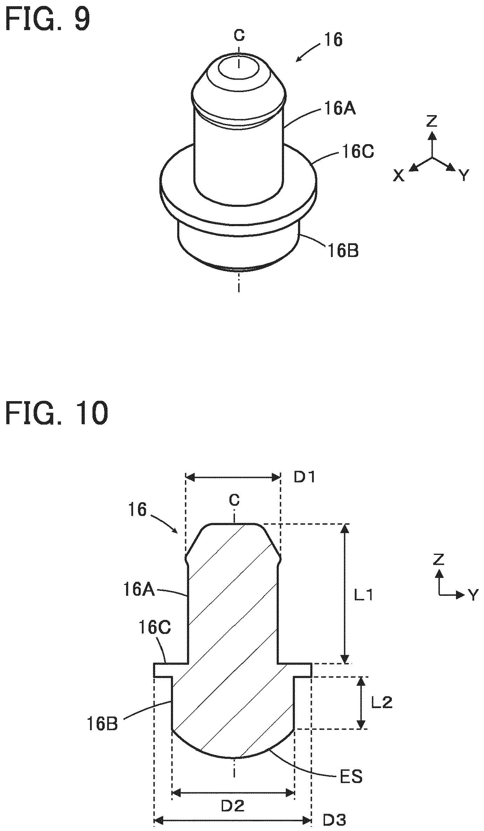

As shown in FIGS. 9 and 10, the conductive member 16 is a solid member made of an electrically conductive material such as metal and includes: a contact portion 16A of substantially round columnar shape that extends in the Z direction along a fitting axis C; an electrode portion 16B of substantially round columnar shape that is adjacent to the -Z direction side of the contact portion 16A and extends in the Z direction along the fitting axis C; and a flange portion 16C of flange shape that overhangs along an XY plane from a border portion between the contact portion 16A and the electrode portion 16B. The conductive member 16 as above can be manufactured by, for instance, press working or cold rolling.

The fitting axis C is an axis passing the center of the conductive member 16 and extending in the fitting direction in which the connector 11 for biological data acquisition is fitted with a counter connector.

Each contact through-hole 13B of the top insulator 13 shown in FIG. 5 has an inside diameter slightly larger than a maximum diameter D1 of the contact portion 16A of the conductive member 16 and smaller than a diameter D3 of the flange portion 16C. A length L1, in the Z direction, of the contact portion 16A of the conductive member 16 is set to be larger than the thickness, in the Z direction, of a bottom portion of the recessed portion 13A of the top insulator 13.

Each electrode through-hole 14B of the bottom insulator 14 shown in FIG. 7 has an inside diameter slightly larger than a diameter D2 of the electrode portion 16B of the conductive member 16 and smaller than the diameter D3 of the flange portion 16C. The electrode portion 16B of the conductive member 16 includes a round columnar shape portion extending in the Z direction by a length L2 along the fitting axis C and a bulge portion bulging in the -Z direction from the round columnar shape portion. The surface of the bulge portion forms the electrode surface ES in a convex shape. The length L2, in the Z direction, of the round columnar shape portion is set to be substantially equal to the thickness, in the Z direction, of the flat plate portion 14A of the bottom insulator 14.

The flange portion 16C of the conductive member 16 has a thickness in the Z direction that is substantially equal to the thickness of the cloth 21.

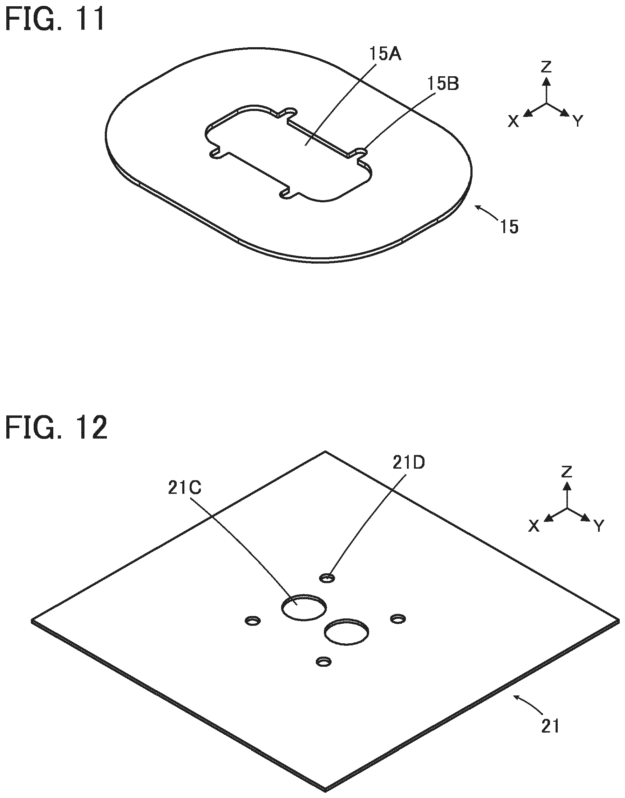

As shown in FIG. 11, the reinforcement sheet 15 is used to reinforce the cloth 21 of the garment on which the connector 11 for biological data acquisition is to be mounted, is made of an insulating material, and has an opening portion 15A formed in its center. Further, four cutouts 15B corresponding to the four bosses 13D of the top insulator 13 are formed along the periphery of the opening portion 15A of the reinforcement sheet 15.

As shown in FIG. 12, the cloth 21 of the garment is provided with two through-holes 21C corresponding to the two conductive members 16 and four through-holes 21D arranged around the two through-holes 21C and corresponding to the four bosses 13D of the top insulator 13.

Each through-hole 21C of the cloth 21 has a diameter substantially equal to or slightly larger than the diameter D3 of the flange portion 16C of the conductive member 16.

The two contact through-holes 13B of the top insulator 13, the two through-holes 21C of the cloth 21, the two conductive members 16, the two electrode through-holes 14B of the bottom insulator 14 are arranged to align correspondingly in the Z direction.

The four bosses 13D of the top insulator 13, the four cutouts 15B of the reinforcement sheet 15, the four through-holes 21D of the cloth 21, and the four boss through-holes 14C of the bottom insulator 14 are arranged to align correspondingly in the Z direction.

When the connector 11 for biological data acquisition is assembled, first, the reinforcement sheet 15 and the cloth 21 are sequentially disposed over the surface, facing in the -Z direction, of the top insulator 13 such that the four bosses 13D of the top insulator 13 pass through the four cutouts 15B of the reinforcement sheet 15 and the four through-holes 21D of the cloth 21. At this time, a peripheral portion of the opening portion 15A of the reinforcement sheet 15 is accommodated in the step portion 13C of the top insulator 13, and the two through-holes 21C of the cloth 21 are situated on the - Z direction side of the two contact through-holes 13B of the top insulator 13 through the opening portion 15A of the reinforcement sheet 15.

Further, the contact portions 16A of the two conductive members 16 are inserted into the two through-holes 21C of the cloth 21 and the two contact through-holes 13B of the top insulator 13 from the -Z direction. The contact through-holes 13B of the top insulator 13 have an inside diameter slightly larger than the diameter D1 of the contact portions 16A of the conductive members 16 and smaller than the diameter D3 of the flange portions 16C, and the through-holes 21C of the cloth 21 have a diameter substantially equal to or slightly larger than the diameter D3 of the flange portions 16C of the conductive members 16.

Accordingly, when the contact portions 16A of the conductive members 16 are inserted into the through-holes 21C of the cloth 21 and the contact through-holes 13B of the top insulator 13, the flange portions 16C of the conductive members 16 abut the surface, facing in the -Z direction, of the top insulator 13 around the contact through-holes 13B.

In this state, while the electrode portions 16B of the two conductive members 16 are inserted into the two electrode through-holes 14B of the bottom insulator 14, the bottom insulator 14 is disposed on the surface, facing in the -Z direction, of the cloth 21 from the -Z direction and pressed in the +Z direction against the top insulator 13.

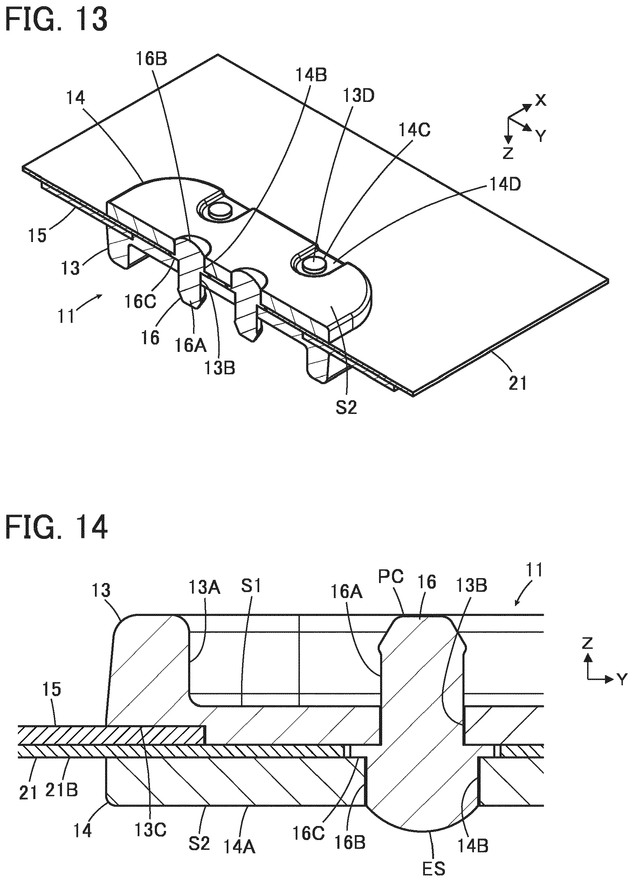

The electrode through-holes 14B of the bottom insulator 14 have an inside diameter slightly larger than the diameter D2 of the electrode portions 16B of the conductive members 16 and smaller than the diameter D3 of the flange portions 16C; accordingly, when the bottom insulator 14 is disposed on the -Z direction side of the cloth 21 while the electrode portions 16B of the conductive members 16 are inserted into the electrode through-holes 14B of the bottom insulator 14, the surface, facing in the +Z direction, of the bottom insulator 14 around the electrode through-holes 14B abuts the flange portions 16C of the conductive members 16 as shown in FIG. 13.

Since the flange portions 16C of the conductive members 16 have a thickness in the Z direction that is substantially equal to the thickness of the cloth 21, the flange portions 16C of the conductive members 16 and the cloth 21 are sandwiched between the surface, facing in the -Z direction, of the top insulator 13 and the surface, facing in the +Z direction, of the bottom insulator 14.

In addition, by pressing the bottom insulator 14 against the top insulator 13, the four bosses 13D of the top insulator 13 sequentially passing through the four cutouts 15B of the reinforcement sheet 15 and the four through-holes 21D of the cloth 21 pass through the four boss through-holes 14C of the bottom insulator 14 and protrude on the -Z direction side of the bottom insulator 14. Then, the tip portions of the four bosses 13D protruding from the bottom insulator 14 on the -Z direction side are thermally deformed in the recessed portions 14D of the bottom insulator 14, whereby the top insulator 13 and the bottom insulator 14 are fixed together. Thus, the assembling operation of the connector 11 for biological data acquisition is completed.

In the thus-assembled connector 11 for biological data acquisition, the electrode portion 16B of each conductive member 16 is inserted in the corresponding electrode through-hole 14B of the bottom insulator 14, and the length of the round columnar shape portion of the electrode portion 16B in the Z direction is substantially equal to the thickness of the flat plate portion 14A of the bottom insulator 14 in the Z direction, as shown in FIG. 14. Consequently, the convex electrode surface ES formed of the surface of the bulge portion adjacent to the -Z direction side of the round columnar shape portion of the conductive member 16 protrudes in the -Z direction from the second exposed surface S2 formed of the surface, facing in the -Z direction, of the bottom insulator 14.

Thus, when a user wears the garment such that the rear surface 21B of the cloth 21 faces inward, and the electrode surfaces ES of the conductive members 16 protruding from the second exposed surface S2 are brought into contact with the body surface of the user, biological data of the user can be acquired via the electrode surfaces ES.

Aside from that, the contact portion 16A of each conductive member 16 passes through the corresponding contact through-hole 13B of the top insulator 13 and protrudes in the +Z direction from the first exposed surface S1 formed of the bottom surface of the recessed portion 13A; thus, the contact portion 16A constitutes the plug contact PC. Therefore, by fitting a counter connector (not shown) to the connector 11 for biological data acquisition such that a part of the counter connector is accommodated in the recessed portion 13A of the top insulator 13 and that counter contacts of the counter connector are connected to the plug contacts PC, biological data of the user acquired through the electrode surfaces ES can be transmitted from the contact portions 16A of the conductive members 16 to a device (not shown) such as a measurement device or a communication device via the counter connector with high accuracy, without use of wiring or the like.

Aside from that, the electrode surface ES used to acquire biological data of the user and the plug contact PC used to transmit the acquired biological data to the counter connector are together formed of a single conductive member 16; therefore, acquisition and transmission of biological data can be carried out only by mounting the connector 11 for biological data acquisition onto the garment, thus making it possible to reduce the size of the device that is mounted on the garment to acquire biological data.

While both the contact portion 16A and the electrode portion 16B of the conductive member 16 have substantially round columnar shapes, it suffices if the portions 16A and 16B have columnar shapes extending in the Z direction along the fitting axis C. The cross-sectional shapes of the portions 16A and 16B when cut in a plane parallel to an XY plane are each not limited to a circular shape and may be an elliptical shape, a rectangular shape, another polygonal shape, or the like.

### Embodiment 2

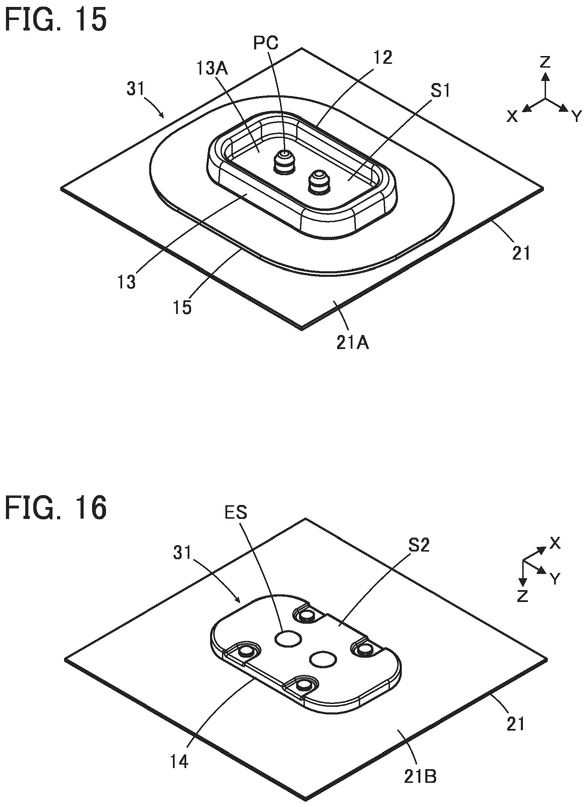

FIGS. 15 and 16 show a connector 31 for biological data acquisition according to Embodiment 2. The connector 31 for biological data acquisition is mounted on the cloth 21 of the garment and used as, for example, a garment-side connector for fitting a wearable device, as with the connector 11 for biological data acquisition according to Embodiment 1.

The connector 31 for biological data acquisition includes the housing 12 made of an insulating material, and the two plug contacts PC are retained in the housing 12, as with the connector 11 for biological data acquisition according to Embodiment 1.

The housing 12 includes the top insulator 13 disposed on the top surface 21A of the cloth 21 and the bottom insulator 14 disposed on the rear surface 21B of the cloth 21.

The two plug contacts PC protrude perpendicularly to the first exposed surface S1 formed of the bottom surface of the recessed portion 13A of the top insulator 13.

The two electrode surfaces ES are disposed and exposed at the second exposed surface S2 formed in the bottom insulator 14.

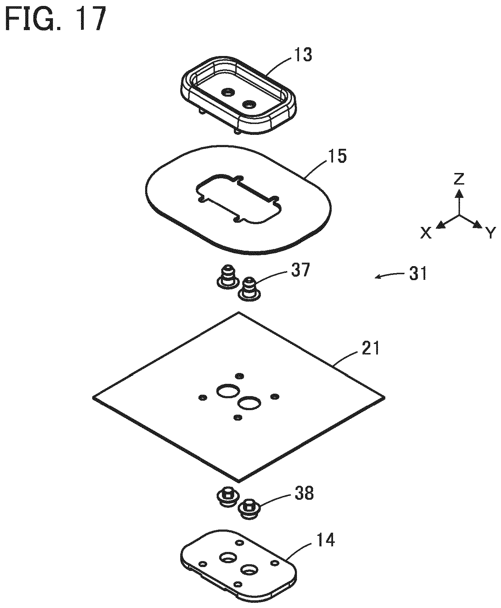

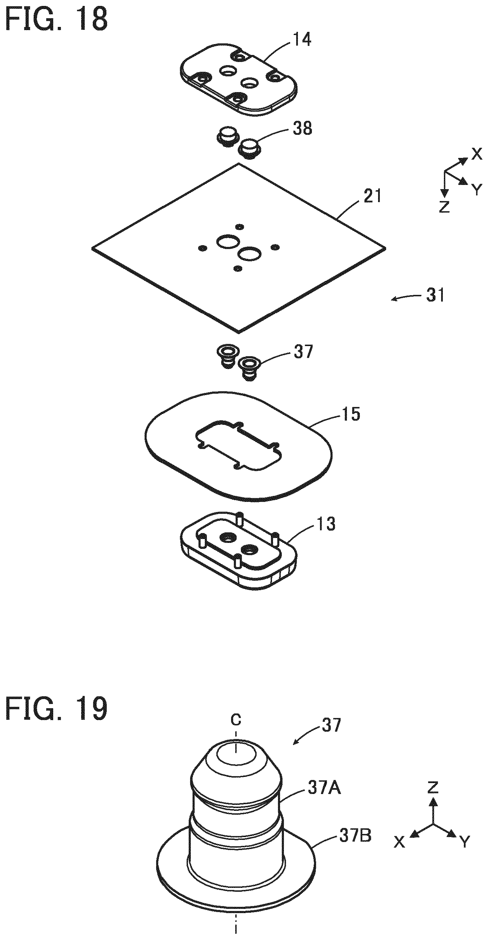

FIGS. 17 and 18 show assembly views of the connector 31 for biological data acquisition. The reinforcement sheet 15 is disposed on the -Z direction side of the top insulator 13, two contact members 37 are disposed on the -Z direction side of the reinforcement sheet 15, and the cloth 21 is disposed on the -Z direction side of the two contact members 37. Further, two electrode members 38 are disposed on the -Z direction side of the cloth 21, and the bottom insulator 14 is disposed on the -Z direction side of the two electrode members 38.

The connector 31 for biological data acquisition according to Embodiment 2 is configured such that, in the connector 11 for biological data acquisition according to Embodiment 1, the two conductive members 16 are replaced by the two contact members 37 and the two electrode members 38, while the top insulator 13, the bottom insulator 14, and the reinforcement sheet 15 are identical to those used in Embodiment 1.

As shown in FIG. 19, the contact member 37 is made of an electrically conductive material such as metal and includes: a tubular portion 37A of cylindrical shape extending in the Z direction along the fitting axis C; and a contact-side flange 37B overhanging from a -Z directional end portion of the tubular portion 37A along an XY plane.

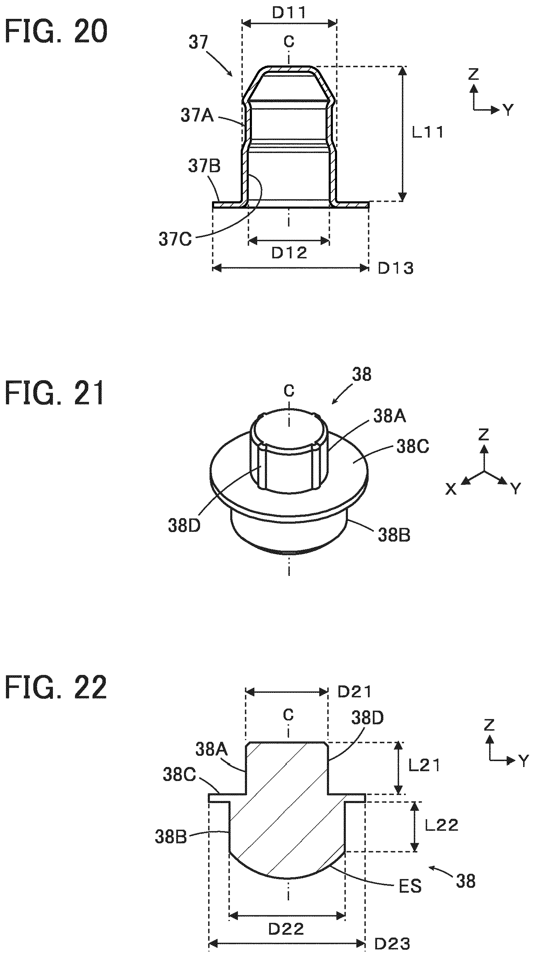

As shown in FIG. 20, the tubular portion 37A is provided in its interior with a recessed portion 37C opening in the -Z direction.

Each contact through-hole 13B of the top insulator 13 shown in FIG. 5 has an inside diameter slightly larger than a maximum diameter D11 of the tubular portion 37A of the contact member 37 and smaller than a diameter D13 of the contact-side flange 37B. A length L11, in the Z direction, of the tubular portion 37A is set to be larger than the thickness, in the Z direction, of the bottom portion of the recessed portion 13A of the top insulator 13.

As shown in FIGS. 21 and 22, the electrode member 38 is a solid member made of an electrically conductive material such as metal and includes: a first round columnar portion 38A of substantially round columnar shape that extends in the Z direction along the fitting axis C; a second round columnar portion 38B of substantially round columnar shape that is adjacent to the -Z direction side of the first round columnar portion 38A and extends in the Z direction along the fitting axis C; and an electrode-side flange 38C of flange shape that overhangs along an XY plane from the border portion between the first round columnar portion 38A and the second round columnar portion 38B.

The first round columnar portion 38A is provided on its outer peripheral portion with four ribs 38D linearly extending in the Z direction and arranged at intervals of 90-degree rotational angles about the fitting axis C.

The electrode member 38 as above can be manufactured by, for instance, press working or cold rolling.

Each electrode through-hole 14B of the bottom insulator 14 shown in FIG. 7 has an inside diameter slightly larger than a diameter D22 of the second round columnar portion 38B of the electrode member 38 and smaller than a diameter D23 of the electrode-side flange 38C. The second round columnar portion 38B of the electrode member 38 includes a round columnar shape portion extending in the Z direction by a length L22 along the fitting axis C and a bulge portion bulging in the -Z direction from the round columnar shape portion. The surface of the bulge portion forms an electrode surface ES in a convex shape. The length L22, in the Z direction, of the round columnar shape portion is set to be substantially equal to the thickness, in the Z direction, of the flat plate portion 14A of the bottom insulator 14.

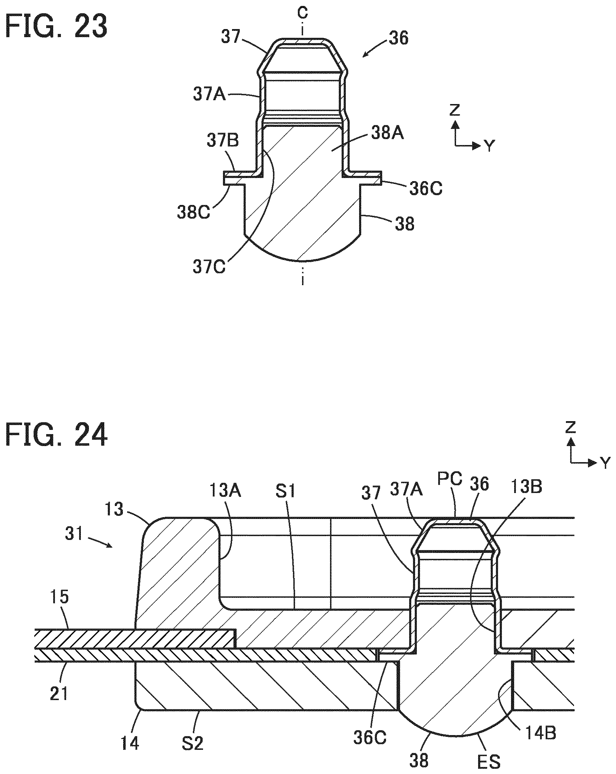

A maximum diameter D21 of the first round columnar portion 38A where the ribs 38D are present is slightly larger than an inside diameter D12 of the recessed portion 37C of the tubular portion 37A of the contact member 37, and a length L21, in the Z direction, of the first round columnar portion 38A is formed to be smaller than a depth, in the Z direction, of the recessed portion 37C of the tubular portion 37A of the contact member 37. Owing to this configuration, when the first round columnar portion 38A of the electrode member 38 is press-fitted into the recessed portion 37C of the tubular portion 37A of the contact member 37 from the -Z direction, the contact member 37 and the electrode member 38 are joined and electrically connected together as shown in FIG. 23, thus forming a conductive member 36 in Embodiment 2.

The electrode member 38 may be provided with a spring portion which is not shown such that the contact member 37 and the electrode member 38 elastically contact each other by use of the spring portion.

The contact member 37 and the electrode member 38 form a contact portion and an electrode portion in the conductive member 36, respectively, and the contact-side flange 37B of the contact member 37 and the electrode-side flange 38C of the electrode member 38 are stacked in the Z direction to form a flange portion 36C in the conductive member 36.

The diameter D13 of the contact-side flange 37B of the contact member 37 and the diameter D23 of the electrode-side flange 38C of the electrode member 38 shown in FIGS. 20 and 22 are equal to each other, and the sum of the thickness of the contact-side flange 37B in the Z direction and the thickness of the electrode-side flange 38C in the Z direction, that is, the thickness of the flange portion 36C in the Z direction is formed to be substantially equal to the thickness of the cloth 21.

When the connector 31 for biological data acquisition is assembled, first, the reinforcement sheet 15 is disposed on the surface, facing in the -Z direction, of the top insulator 13 such that the four bosses 13D of the top insulator 13 separately pass through the four cutouts 15B of the reinforcement sheet 15, and the tubular portions 37A of the two contact members 37 are inserted into the two contact through-holes 13B of the top insulator 13 through the opening portion 15A of the reinforcement sheet 15 from the -Z direction.

Subsequently, the cloth 21 is disposed on the surface, facing in the -Z direction, of the reinforcement sheet 15 such that the four bosses 13D of the top insulator 13 separately pass through the four through-holes 21D of the cloth 21, and the first round columnar portions 38A of the two electrode members 38 are press-fitted into the recessed portions 37C of the two contact members 37 through the two through-holes 21C of the cloth 21 from the -Z direction. Thus, the two conductive members 36 each of which is as shown in FIG. 23 are formed.

In this state, while the second round columnar portions 38B of the two electrode members 38 each being a constituent of the conductive member 36 are inserted into the two electrode through-holes 14B of the bottom insulator 14, the bottom insulator 14 is disposed on the surface, facing in the -Z direction, of the cloth 21 from the -Z direction and pressed in the +Z direction against the top insulator 13.

Then, the tip portions of the four bosses 13D of the top insulator 13 passing through the four boss through-holes 14C of the bottom insulator 14 and protruding from the bottom insulator 14 on the -Z direction side are thermally deformed, whereby the top insulator 13 and the bottom insulator 14 are fixed together. Thus, the assembling operation of the connector 31 for biological data acquisition is completed.

In the thus-assembled connector 31 for biological data acquisition, the flange portion 36C of the conductive member 36 is sandwiched between the surface, facing in the -Z direction, of the top insulator 13 and the surface, facing in the +Z direction, of the bottom insulator 14, so that the conductive member 36 is fixed to the top insulator 13 and the bottom insulator 14, as shown in FIG. 24.

The convex electrode surface ES of the electrode member 38 that is a constituent of each conductive member 36 protrudes in the -Z direction from the second exposed surface S2 formed of the surface, facing in the -Z direction, of the bottom insulator 14.

The tubular portion 37A of the contact member 37 that is a constituent of each conductive member 36 passes through the contact through-hole 13B of the top insulator 13 and protrudes in the +Z direction from the first exposed surface S1 formed of the bottom surface of the recessed portion 13A; thus, the tubular portion 37A constitutes the plug contacts PC.

Therefore, also in the connector 31 for biological data acquisition, by contact of the electrode surfaces ES protruding from the second exposed surface S2 with the body surface of a user, biological data of the user can be acquired via the electrode surface ES as with the connector 11 for biological data acquisition according to Embodiment 1. Furthermore, when a counter connector (not shown) is fitted to the connector 31 for biological data acquisition such that counter contacts of the counter connector are connected to the plug contacts PC, biological data of the user acquired through the electrode surfaces ES can be transmitted from the contact members 37 of the conductive members 36 to a device (not shown) such as a measurement device or a communication device via the counter connector with high accuracy, without use of wiring or the like.

Aside from that, both the electrode surface ES used to acquire biological data of the user and the plug contact PC used to transmit the acquired biological data to the counter connector are formed of a single conductive member 36; therefore, acquisition and transmission of biological data can be carried out only by mounting the connector 31 for biological data acquisition onto the garment, thus making it possible to reduce the size of the device that is mounted on the garment to acquire biological data.

The tubular portion 37A of the contact member 37 has a cylindrical shape, and the first round columnar portion 38A and the second round columnar portion 38B of the electrode member 38 have substantially round columnar shapes; however, it suffices if the portions 37A, 38A, and 38B each have a columnar shape extending in the Z direction along the fitting axis C. The cross-sectional shapes of the portions 37A, 38A, and 38B when cut in a plane parallel to an XY plane are each not limited to a circular shape and may be an elliptical shape, a rectangular shape, another polygonal shape, or the like.

Nevertheless, since the first round columnar portion 38A of the electrode member 38 is press-fitted into the recessed portion 37C of the tubular portion 37A of the contact member 37, it is desirable that the cross-sectional shape of the first round columnar portion 38A and that of the recessed portion 37C correspond to each other.

In Embodiment 2, since the contact member 37 and the electrode member 38 are configured to be separate components, it is not necessary to form the contact member 37 and the electrode member 38 from the same material. For instance, the contact member 37 may be formed from a material suitable for contact with a counter contact of a counter connector, while the electrode member 38 may be formed from a material suitable for contact with the body surface of a user.

Various types of biological data of a user can be acquired by contact of the electrode surfaces ES of the conductive members 16, 36 in Embodiments 1 and 2 with the body surface of the user, and the connector 11, 31 for biological data acquisition according to Embodiments 1 and 2 has the two conductive members 16, 36. Therefore, the use of only the connector 11, 31 for biological data acquisition allows acquisition of even a heart rate, an electrocardiographic waveform, and other types of biological data with high accuracy. However, the number of conductive members is not limited to two, and a connector for biological data acquisition may be configured to have three or more conductive members.

While, in Embodiments 1 and 2, the electrode portion 16B of the conductive member 16 and the electrode member 38 of the conductive member 36 each of which has the electrode surface ES protruding from the second exposed surface S2 are made of metal or the like, the invention is not limited thereto, and the electrode portion 16B and the electrode member 38 may be formed from a conductive material having flexibility such as conductive rubber.

### Embodiment 3

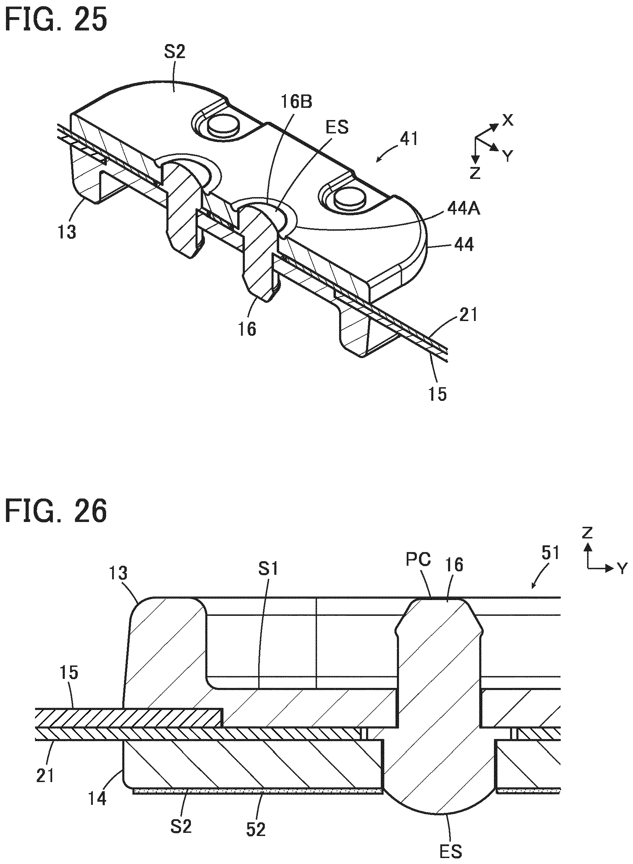

FIG. 25 shows a connector 41 for biological data acquisition according to Embodiment 3. The connector 41 for biological data acquisition is configured such that, in the connector 11 for biological data acquisition according to Embodiment 1, the bottom insulator 14 is replaced by a bottom insulator 44, and otherwise has the same configuration as the connector 11 for biological data acquisition according to Embodiment 1.

Also in the connector 41 for biological data acquisition, the electrode surfaces ES of the two conductive members 16 are exposed in the state of protruding in the -Z direction from the second exposed surface S2 of the bottom insulator 44, as with the connector 11 for biological data acquisition according to Embodiment 1. The bottom insulator 44 has lubricant retaining portions 44A constituted of annular grooves separately formed along the outer peripheries of the two electrode surfaces ES exposed at the second exposed surface S2. The bottom insulator 44 has the same configuration as that of the bottom insulator 14 in Embodiment 1 except for the provision of those lubricant retaining portions 44A.

To acquire biological data of a user via the electrode surfaces ES, it is desirable that the electrode surfaces ES closely adhere to the body surface of the user, and for instance, a medical lubricant may be applied on the body surface of the user to contact the electrode surfaces ES with the body surface.

Since the lubricant retaining portions 44A constituted of grooves are formed along the outer peripheries of the two electrode surfaces ES exposed at the second exposed surface S2 of the bottom insulator 44 in the connector 41 for biological data acquisition according to Embodiment 3, when the electrode surfaces ES are brought into contact with the body surface of the user, the medical lubricant applied on the body surface of the user is retained inside the lubricant retaining portions 44A.

Even when no medical lubricant is used, moisture such as sweat is retained in the lubricant retaining portion 44A and supplied between the electrode surface ES and the body surface of the user, so that the adhesion is maintained between the electrode surface ES and the body surface of the user, thus enabling to acquire biological data with high accuracy.

The lubricant retaining portions 44A in Embodiment 3 may be adopted for the connector 31 for biological data acquisition according to Embodiment 2 to be formed in the bottom insulator 14 of the connector 31 for biological data acquisition.

### Embodiment 4

FIG. 26 shows a connector 51 for biological data acquisition according to Embodiment 4. The connector 51 for biological data acquisition is configured such that, in the connector 11 for biological data acquisition according to Embodiment 1, a pressure-sensitive adhesive layer 52 is disposed on the second exposed surface S2, facing in the -Z direction, of the bottom insulator 14, and otherwise has the same configuration as the connector 11 for biological data acquisition according to Embodiment 1.

The pressure-sensitive adhesive layer 52 is used to adhere the connector 51 for biological data acquisition to the body surface of a user by utilizing its adhesion and can be formed using a conventional elastomer such as silicone rubber or urethane rubber.

By adhering the connector 51 for biological data acquisition to the body surface of the user by use of the pressure-sensitive adhesive layer 52, the adhesion between the electrode surfaces ES and the body surface of the user enhances, thus enabling to acquire biological data via the electrode surfaces ES with high accuracy.

The pressure-sensitive adhesive layer 52 in Embodiment 4 may be adopted for the connector 31 for biological data acquisition according to Embodiment 2 or the connector 41 for biological data acquisition according to Embodiment 3.

Besides, the bottom insulator 14 in the connector 11 for biological data acquisition according to Embodiment 1 and the connector 31 for biological data acquisition according to Embodiment 2 and the bottom insulator 44 in the connector 41 for biological data acquisition according to Embodiment 3 may be formed from an insulating material having pressure-sensitive adhesive properties. For the insulating material having pressure-sensitive adhesive properties, use may be made of, for example, an elastomer including silicone rubber or urethane rubber.

When the bottom insulator 14, 44 is formed from such an insulating material having pressure-sensitive adhesive properties, the connector 11, 31, 41 for biological data acquisition adheres to the body surface of the user, and this enhances the adhesion between the electrode surfaces ES and the body surface of the user, thus enabling to acquire biological data via the electrode surfaces ES with high accuracy.

Besides, the top and bottom insulators 13 and 14 in the connector 11 for biological data acquisition according to Embodiment 1, the connector 31 for biological data acquisition according to Embodiment 2, and the connector 51 for biological data acquisition according to Embodiment 4 and the top and bottom insulators 13 and 44 in the connector 41 for biological data acquisition according to Embodiment 3 may be formed from an insulating material having flexibility such as an elastomer. When the top insulator 13 and the bottom insulator 14, 44 as above are used, the connector 11, 31, 41, 51 for biological data acquisition can be bent to follow the body surface of a user, and this further enhances the adhesion between the electrode surfaces ES and the body surface of the user.

In the case where a connector for biological data acquisition having a large number of conductive members 16, 36 is configured, it is necessary to increase the area of the top insulator 13 and the bottom insulator 14, 44 along an XY plane in accordance with the number of the conductive members 16, 36; even in this case, when the top insulator 13 and the bottom insulator 14, 44 are formed from an insulating material having flexibility, the adhesion between the electrode surfaces ES of the respective conductive members 16, 36 and the body surface of a user can be ensured, thus enabling to acquire biological data with high accuracy.

Alternatively, the electrode surfaces ES of the respective conductive members 16, 36 may be provided with asperities including, for example, grooves and the like to roughen the electrode surfaces ES. When the electrode surfaces ES are formed as above, a medical lubricant or moisture such as sweat is more likely to remain on the electrode surfaces ES, and this enhances the adhesion between the electrode surfaces ES and the body surface of a user, thus enabling to acquire biological data with high accuracy.

### Embodiment 5

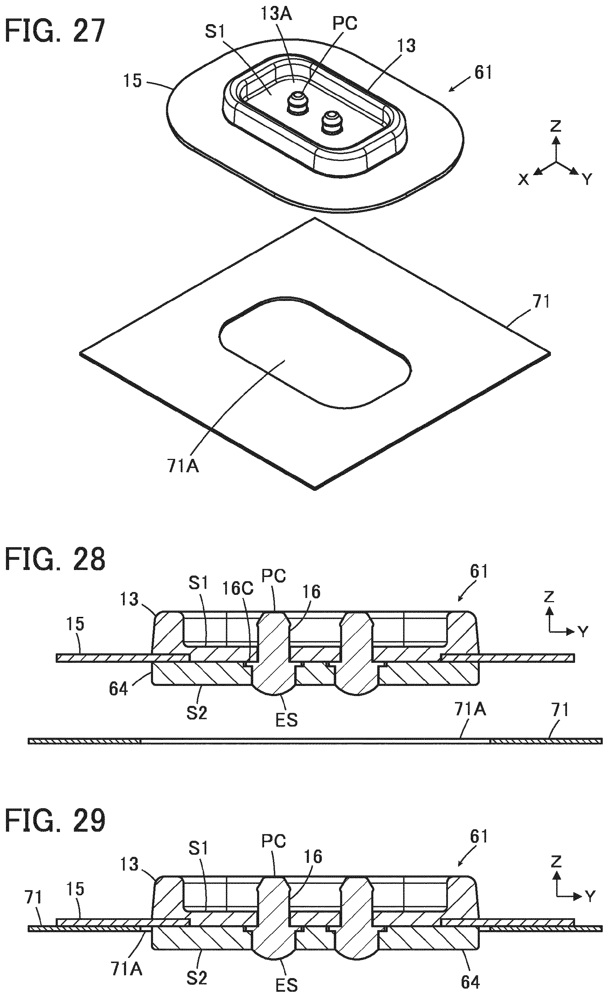

FIG. 27 shows a connector 61 for biological data acquisition according to Embodiment 5 and cloth 71 of a garment that serves as a mounting object.

As with the connector 11 for biological data acquisition according to Embodiment 1, the connector 61 for biological data acquisition includes: the top insulator 13 disposed on the +Z directional surface of the reinforcement sheet 15; and the two plug contacts PC protruding in the +Z direction from the first exposed surface S1 formed of the bottom surface of the recessed portion 13A of the top insulator 13.

However, the connector 61 for biological data acquisition is not retained by the cloth 71 of the garment, and the cloth 71 is disposed away in the -Z direction from the connector 61 for biological data acquisition. The cloth 71 is provided with an opening portion 71A corresponding to the connector 61 for biological data acquisition.

As shown in FIG. 28, the connector 61 for biological data acquisition includes a bottom insulator 64 disposed on the -Z direction side of the reinforcement sheet 15. The +Z directional surface of the bottom insulator 64 makes contact with the -Z directional surface of the reinforcement sheet 15 and the -Z directional surface of the top insulator 13, and the flange portions 16C of the two conductive members 16 are sandwiched between the top insulator 13 and the bottom insulator 64; in this state, the top insulator 13 and the bottom insulator 64 are fixed to each other.

As with the connector 11 for biological data acquisition according to Embodiment 1, the two conductive members 16 form the two plug contacts PC and the two electrode surfaces ES, the two plug contacts PC protrude in the +Z direction from the first exposed surface S1 of the top insulator 13, and the two electrode surfaces ES protrude in the -Z direction from the second exposed surface S2 of the bottom insulator 64.

The connector 61 for biological data acquisition as above is disposed on the cloth 71 of the garment as shown in FIG. 29. The opening portion 71A of the cloth 71 is slightly larger in size than the bottom insulator 64. The bottom insulator 64 is inserted in the opening portion 71A, and the reinforcement sheet 15 makes contact with the surface of the cloth 71 around the opening portion 71A.

In this state, for example, the reinforcement sheet 15 is sewn to the cloth 71, whereby the connector 61 for biological data acquisition can be mounted on the cloth 71.

With the use of the connector 61 for biological data acquisition according to Embodiment 5, it is not necessary to sandwich the cloth 71 between the top insulator 13 and the bottom insulator 64 in the assembling operation of the connector 61 for biological data acquisition, and the connector 61 for biological data acquisition can be mounted on the cloth 71 after the connector 61 for biological data acquisition is assembled.

Accordingly, by mounting the connector 61 for biological data acquisition on a usual or general garment, the garment can be used as so-called smart clothes that can acquire biological data of a user only be being worn by the user. When the garment used as smart clothes has deteriorated, the connector 61 for biological data acquisition can be removed and attached to another garment for reuse.

For the method of fixing the reinforcement sheet 15 to the cloth 71, various methods may be used such as bonding and fixing by means of a zip fastener, in addition to sewing.

Also in Embodiment 5, the top insulator 13 and the bottom insulator 64 in the connector 61 for biological data acquisition may be formed separately from an insulating material having flexibility, such as an elastomer, to make the connector 61 for biological data acquisition bendable to follow the body surface of a user.

Since the connector 61 for biological data acquisition can be mounted on the cloth 71 by fixing the reinforcement sheet 15 to the cloth 71 after the connector 61 for biological data acquisition is assembled in Embodiment 5, the two conductive members 16 may be formed by insert-molding, while the top insulator 13, the bottom insulator 64, and the reinforcement sheet 15 are integrally formed from an insulating material. In this case, the connector 61 for biological data acquisition that is bendable may be formed by molding using an insulating material having flexibility such as an elastomer.

The connector 31 for biological data acquisition according to Embodiment 2, the connector 41 for biological data acquisition according to Embodiment 3, and the connector 51 for biological data acquisition according to Embodiment 4 may also be configured to be mounted on cloth after the connector is assembled, in the same manner.

## Claims

1. A connector (11,31,41,5,61) for biological data acquisitionthat is configured to be mounted on a mounting object (21,71) constituted of a wearing article worn by a user and is configured to be fitted to a counter connector along a fitting direction, the connector (11,31,41,51,61) for biological data acquisition comprising:
a housing (12) configured to be attached to the mounting object (21,71) and including a first exposed surface (S1) that faces an opposite side from a body surface of the user and a second exposed surface (S2) that faces the body surface; and
a plurality of conductive members (16, 36) each extending along the fitting direction and retained by the housing (12) in a state of passing through the housing (12) in the fitting direction from the first exposed surface (S1) to the second exposed surface (S2),
wherein each of the plurality of conductive members (16,36) includes a contact portion (16A, 37) having electrical conductivity and an electrode portion (16B, 38) having electrical conductivity, the contact portion (16A, 37) protruding from the first exposed surface (S1) of the housing (12) and being configured to be connected to a counter contact of the counter connector when the connector (11, 31, 41, 51, 61) for biological data acquisition is fitted to the counter connector, the electrode portion (16B, 38) protruding from the second exposed surface (S2) of the housing (12) and having an electrode surface (ES) which is configured to be brought into contact with the body surface of the user and to acquire biological data of the user,
wherein one of the following two options I and II applies:
option I:
each of the plurality of conductive members (16) is constituted of a single component in which the contact portion (16A) and the electrode portion (16B) are formed to be integral with each other along the fitting direction, and
each of the contact portion (16A) and the electrode portion (16B) has a columnar shape extending along the fitting direction,
wherein each of the plurality of conductive members (16) has a flange portion (16C) overhanging in a direction perpendicular to the fitting direction from a border portion between the contact portion (16A) and the electrode P portion (16B),
wherein the housing (12) is composed of a top insulator (13) having a recessed portion (13A) configured to accommodate the counter connector, the first exposed surface (S1) of the housing (12) being formed by the bottom surface of the recessed portion (13A), and a bottom insulator (14, 44, 64) having the second exposed surface (S2),
the top insulator (13) includes a plurality of contact through-holes (13B) formed within the recessed portion (13A) each of which being configured to receive the contact portion (16A) passing therethrough and is smaller in size than the flange portion (16C),
the bottom insulator (14, 44, 64) includes a plurality of electrode through-holes (14B) each of which being configured to receive the electrode portion (16B) passing therethrough and is smaller in size than the flange portion (16C), and
the top insulator (13) and the bottom insulator (14, 44, 64) are configured to be fixed together in a state where the contact portions (16A) of the plurality of conductive members (16) pass through the plurality of contact through-holes (13B), the electrode portions (16B) of the plurality of conductive member (16) pass through the plurality of electrode through-holes (14B), and the flange portions (16C) of the plurality of conductive members (16) are sandwiched between the top insulator (13) and the bottom insulator (14);
option II:
each of the plurality of conductive members (36) is composed of a contact member (37) that forms the contact portion and an electrode member (38) that is configured to be joined with the contact member (37) and forms the electrode portion, the contact member (37) has a tubular portion (37A) extending along the fitting direction and provided in its interior with a recessed portion (37C), and
a part of the electrode member (38) is configured to be inserted in the recessed portion (37C) and to make contact with an inner surface of the recessed portion (37C), whereby the electrode member (38) i configured to be electrically connected to the contact member (37),
wherein the contact member (37) includes a contact-side flange (37B) overhanging in a direction perpendicular to the fitting direction from an end of the tubular portion (37A),
the electrode member (38) includes a first round columnar portion (38A) that extends along the fitting direction and is configured to be inserted in the recessed portion (37C) of the contact member (37) as the part of the electrode member (38), a second round columnar portion (38B) that extends along the fitting direction in a direction opposite from the first round columnar portion (38A), and an electrode-side flange (38C) overhanging in a direction perpendicular to the fitting direction from a border portion between the first round columnar portion (38A) and the second round columnar portion (38B), and
the contact-side flange (37B) and the electrode-side flange (38C) are configured to be stacked in the fitting direction to form a flange portion (36C),
wherein the housing (12) is composed of a top insulator (13) having a recessed portion (13A) configured to accommodate the counter connector, the first exposed surface (S1) of the housing (12) being formed by the bottom surface of the recessed portion (13A), and a bottom insulator (14) having the second exposed surface (S2),
the top insulator (13) includes a plurality of contact through-holes (13B) formed within the recessed portion (13A) each of which being configured to receive the tubular portion (37A) of the contact member (37) passing therethrough and is smaller in size than the contact-side flange (37B),
the bottom insulator (14) includes a plurality of electrode through-holes (14B) each of which being configured to receive the second round columnar portion (38B) of the electrode member (38) passing therethrough and is smaller in size than the electrode-side flange (38C), and
the top insulator (13) and the bottom insulator (14) are configured to be fixed together in a state where the tubular portions (37A) of the contact members (37) of the plurality of conductive members (16) pass through the plurality of contact through-holes (13B), elem the second round columnar portions (38B) of the electrode members (38) of the plurality of conductive members (16) pass through the plurality of electrode through-holes (14B), and the flange portions (36C) of the plurality of conductive members (16) are sandwiched between the top insulator (13) and the bottom insulator (14).

2. The connector for biological data acquisition according to claim 1,
wherein the top insulator (13) and the bottom insulator (14) are configured to be fixed together with the mounting object (21, 71) being sandwiched between the top insulator (13) and the bottom insulator (14), whereby the housing (12) is attached to the mounting object (21, 71).

3. The connector for biological data acquisition according to any one of claims 1-2, comprising a reinforcement sheet (15) that is retained by the housing (12) and has a fixing portion overhanging outward from an outer peripheral portion of the housing,
wherein the fixing portion is fixed to the mounting object, whereby the housing is attached to the mounting object.

4. The connector for biological data acquisition according to claim 3,
wherein the housing (12) and the reinforcement sheet (15) are integrally formed from insulating resin.

5. The connector for biological data acquisition according to any one of claims 1-4,
wherein the housing (12) includes a lubricant retaining portion (44A) constituted of a groove that is formed at the second exposed surface (S2) and disposed around the electrode portion protruding from the second exposed surface.

6. The connector for biological data acquisition according to any one of claims 1-5, comprising a pressure-sensitive adhesive layer (52) disposed on the second exposed surface (S2).

7. The connector for biological data acquisition according to claim 1,
wherein the bottom insulator (14) is formed from an elastomer.

8. The connector for biological data acquisition according to claim 4,
wherein the insulating resin integrally forming the housing (12) and the reinforcement sheet (15) is constituted of an elastomer.

9. The connector for biological data acquisition according to any one of claims 1-8,
wherein the electrode portion includes an electrode surface (ES) that is exposed from the second exposed surface (S2) and roughened.

10. The connector for biological data acquisition according to any one of claims 1-9 being configured to be mounted on a garment that is the mounting object.

## Patentansprüche

1. Verbinder (11, 31, 41, 51, 61) zur Erfassung biologischer Daten, der eingerichtet ist, um auf einem Montageobjekt (21, 71) montiert zu sein, das aus einem von einem Benutzer getragenen Bekleidungsartikel besteht, und eingerichtet ist, um an einem Gegenverbinder entlang einer Einpassrichtung eingepasst zu sein, wobei der Verbinder (11, 31, 41, 51, 61) zur Erfassung biologischer Daten umfasst:
ein Gehäuse (12), das eingerichtet ist, um an dem Montageobjekt (21, 71) angebracht zu sein, und das eine erste freiliegende Fläche (S1), die von einer Körperfläche des Benutzers abgewandt ist, und eine zweite freiliegende Fläche (S2), die der Körperfläche zugewandt ist, aufweist; und
mehrere leitfähige Elemente (16, 36), die sich jeweils entlang der Einpassrichtung erstrecken und von dem Gehäuse (12) in einem Zustand gehalten sind, in dem sie durch das Gehäuse (12) in der Einpassrichtung von der ersten freiliegenden Fläche (S1) zu der zweiten freiliegenden Fläche (S2) verlaufen,
wobei jedes der mehreren leitenden Elemente (16, 36) einen Kontaktabschnitt (16A, 37) mit elektrischer Leitfähigkeit und einen Elektrodenabschnitt (16B, 38) mit elektrischer Leitfähigkeit aufweist, wobei der Kontaktabschnitt (16A, 37) von der ersten freiliegenden Fläche (S1) des Gehäuses (12) vorsteht und eingerichtet ist, um mit einem Gegenkontakt des Gegenverbinders verbunden zu sein, wenn der Verbinder (11, 31, 41, 51, 61) zur Erfassung biologischer Daten an den Gegenverbinder angebracht ist, wobei der Elektrodenabschnitt (16B, 38) von der zweiten freiliegenden Fläche (S2) des Gehäuses (12) vorsteht und eine Elektrodenfläche (ES) aufweist, die eingerichtet ist, um mit der Körperfläche des Benutzers in Kontakt gebracht zu werden und um biologische Daten des Benutzers zu erfassen,
wobei eine der folgenden zwei Optionen I und II gilt:
Option I:
jedes der mehreren leitfähigen Elemente (16) besteht aus einer einzigen Komponente, bei der der Kontaktabschnitt (16A) und der Elektrodenabschnitt (16B) so gebildet sind, dass sie entlang der Einpassrichtung einstückig miteinander sind, und
sowohl der Kontaktabschnitt (16A) als auch der Elektrodenabschnitt (16B) weisen eine säulenartige Form auf, die sich entlang der Einpassrichtung erstreckt,
wobei jedes der mehreren leitfähigen Elemente (16) einen Flanschabschnitt (16C) aufweist, der in einer Richtung senkrecht zu der Einpassrichtung von einem Grenzabschnitt zwischen dem Kontaktabschnitt (16A) und dem Elektrodenabschnitt (16B) überhängt,
wobei das Gehäuse (12) aus einem oberen Isolator (13) mit einem vertieften Abschnitt (13A), der eingerichtet ist, um den Gegenverbinder aufzunehmen, wobei die erste freiliegende Fläche (S1) des Gehäuses (12) von der Bodenfläche des vertieften Abschnitts (13A) gebildet ist, und einem unteren Isolator (14, 44, 64) mit der zweiten freiliegenden Fläche (S2) besteht,
der obere Isolator (13) weist mehrere Kontakt-Durchgangslöcher (13B) auf, die innerhalb des vertieften Abschnitts (13A) gebildet sind, von denen jedes eingerichtet ist, um den hindurchgehenden Kontaktabschnitt (16A) aufzunehmen, und eine kleinere Größe als der Flanschabschnitt (16C) aufweist,
der untere Isolator (14, 44 64) weist mehrere Elektroden-Durchgangslöcher (14B) auf, von denen jedes eingerichtet ist, um den hindurchgehenden Elektrodenabschnitt (16B) aufzunehmen, und eine kleinere Größe als der Flanschabschnitt (16C) aufweist, und
der obere Isolator (13) und der untere Isolator (14, 44, 64) sind eingerichtet, um in einem Zustand aneinander befestigt zu sein, in dem die Kontaktabschnitte (16A) der mehreren leitfähigen Elemente (16) durch die mehreren Kontakt-Durchgangslöcher (13B) verlaufen, die Elektrodenabschnitte (16B) der mehreren leitfähigen Elemente (16) durch die mehreren Elektroden-Durchgangslöcher (14B) verlaufen und die Flanschabschnitte (16C) der mehreren leitfähigen Elemente (16) sandwichartig zwischen dem oberen Isolator (13) und dem unteren Isolator (14) aufgenommen sind;
Option II:
jedes der mehreren leitfähigen Elemente (36) besteht aus einem Kontaktelement (37), das den Kontaktabschnitt bildet, und einem Elektrodenelement (38), das eingerichtet ist, um mit dem Kontaktelement (37) verbunden zu sein, und bildet den Elektrodenabschnitt,
das Kontaktelement (37) weist einen röhrenförmigen Abschnitt (37A) auf, der sich entlang der Einpassrichtung erstreckt und in seinem Inneren mit einem vertieften Abschnitt (37C) versehen ist, und
ein Teil des Elektrodenelements (38) ist eingerichtet, um in den vertieften Abschnitt (37C) eingesetzt zu sein und mit einer inneren Fläche des vertieften Abschnitts (37C) Kontakt herzustellen, wodurch das Elektrodenelement (38) eingerichtet ist, um mit dem Kontaktelement (37) elektrisch verbunden zu sein,
wobei das Kontaktelement (37) einen kontaktseitigen Flansch (37B) aufweist, der in einer Richtung senkrecht zu der Einpassrichtung von einem Ende des röhrenförmigen Abschnitts (37A) überhängt,
das Elektrodenelement (38) weist einen ersten rundsäulenartigen Abschnitt (38A), der sich entlang der Einpassrichtung erstreckt und eingerichtet ist, um in den vertieften Abschnitt (37C) des Kontaktelements (37) als der Teil des Elektrodenelements (38) eingesetzt zu sein, einen zweiten rundsäulenartigen Abschnitt (38B), der sich entlang der Einpassrichtung in einer Richtung entgegengesetzt zu dem ersten rundsäulenartigen Abschnitt (38A) erstreckt, und einen elektrodenseitigen Flansch (38C), der in einer Richtung senkrecht zu der Einpassrichtung von einem Grenzabschnitt zwischen dem ersten rundsäulenartigen Abschnitt (38A) und dem zweiten rundsäulenartigen Abschnitt (38B) überhängt, auf und
der kontaktseitige Flansch (37B) und der elektrodenseitige Flansch (38C) sind eingerichtet, um in der Einpassrichtung gestapelt zu sein, um einen Flanschabschnitt (36C) zu bilden,
wobei das Gehäuse (12) aus einem oberen Isolator (13) mit einem vertieften Abschnitt (13A), der eingerichtet ist, um den Gegenverbinder aufzunehmen, wobei die erste freiliegende Fläche (S1) des Gehäuses (12) von der Bodenfläche des vertieften Abschnitts (13A) gebildet ist, und einem unteren Isolator (14) mit der zweiten freiliegenden Fläche (S2) besteht,
der obere Isolator (13) weist mehrere Kontakt-Durchgangslöcher (13B) auf, die innerhalb des vertieften Abschnitts (13A) gebildet sind, von denen jedes eingerichtet ist, um den röhrenförmigen Abschnitt (37A) des hindurchgehenden Kontaktelements (37) aufzunehmen, und eine kleinere Größe als der kontaktseitige Flansch (37B) aufweist,
der untere Isolator (14) weist mehrere Elektroden-Durchgangslöcher (14B) auf, von denen jedes eingerichtet ist, um den zweiten rundsäulenartigen Abschnitt (38B) des hindurchgehenden Elektrodenelements (38) aufzunehmen, und eine kleinere Größe als der elektrodenseitige Flansch (38C) aufweist, und
der obere Isolator (13) und der untere Isolator (14) sind eingerichtet, um in einem Zustand aneinander befestigt zu sein, in dem die röhrenförmigen Abschnitte (37A) der Kontaktelemente (37) der mehreren leitfähigen Elemente (16) durch die mehreren Kontakt-Durchgangslöcher (13B) verlaufen, die zweiten rundsäulenartigen Abschnitte (38B) der Elektrodenelemente (38) der mehreren leitfähigen Elemente (16) durch die mehreren Elektroden-Durchgangslöcher (14B) verlaufen und die Flanschabschnitte (36C) der mehreren leitfähigen Elemente (16) sandwichartig zwischen dem oberen Isolator (13) und dem unteren Isolator (14) aufgenommen sind.

2. Verbinder zur Erfassung biologischer Daten nach Anspruch 1,
wobei der obere Isolator (13) und der untere Isolator (14) eingerichtet sind, um zusammen mit dem Montageobjekt (21, 71) befestigt zu sein, das sandwichartig zwischen dem oberen Isolator (13) und dem unteren Isolator (14) aufgenommen ist, wodurch das Gehäuse (12) an dem Montageobjekt (21, 71) angebracht ist.

3. Verbinder zur Erfassung biologischer Daten nach einem der Ansprüche 1-2, umfassend eine Verstärkungsplatte (15), die von dem Gehäuse (12) gehalten ist und einen Befestigungsabschnitt aufweist, der von einem äußeren Umfangsabschnitt des Gehäuses nach außen überhängt,
wobei der Befestigungsabschnitt an dem Montageobjekt befestigt ist, wodurch das Gehäuse an dem Montageobjekt angebracht ist.

4. Verbinder zur Erfassung biologischer Daten nach Anspruch 3,
wobei das Gehäuse (12) und die Verstärkungsplatte (15) einstückig aus Isolierharz gebildet sind.

5. Verbinder zur Erfassung biologischer Daten nach einem der Ansprüche 1-4,
wobei das Gehäuse (12) einen Schmiermittelhalteabschnitt (44A) aufweist, der aus einer Nut besteht, die an der zweiten freiliegenden Fläche (S2) gebildet ist und um den Elektrodenabschnitt herum angeordnet ist, der von der zweiten freiliegenden Fläche vorsteht.

6. Verbinder zur Erfassung biologischer Daten nach einem der Ansprüche 1-5, umfassend eine druckempfindliche Haftschicht (52), die an der zweiten freiliegenden Fläche (S2) angeordnet ist.

7. Verbinder zur Erfassung biologischer Daten nach Anspruch 1,
wobei der untere Isolator (14) aus einem Elastomer gebildet ist.

8. Verbinder zur Erfassung biologischer Daten nach Anspruch 4,
wobei das Isolierharz, das das Gehäuse (12) und die Verstärkungsplatte (15) einstückig bildet, aus einem Elastomer besteht.

9. Verbinder zur Erfassung biologischer Daten nach einem der Ansprüche 1-8,
wobei der Elektrodenabschnitt eine Elektrodenfläche (ES) aufweist, die von der zweiten freiliegenden Fläche (S2) freiliegend und aufgeraut ist.

10. Verbinder zur Erfassung biologischer Daten nach einem der Ansprüche 1-9, der eingerichtet ist, um auf einem Kleidungsstück montiert zu sein, welches das Montageobjekt ist.

## Revendications

1. Connecteur (11, 31, 41, 51, 61) pour l'acquisition de données biologiques qui est configuré pour être monté sur un objet de montage (21, 71) constitué d'un article de vestimentaire porté par un utilisateur et qui est configuré pour être accouplé sur un contre-connecteur le long d'une direction d'accouplement, le connecteur (11, 31, 41, 51, 61) pour l'acquisition de données biologiques comprenant :
un boîtier (12) configuré pour être attaché à l'objet de montage (21, 71) et comportant une première surface exposée (S1) qui fait face à un côté opposé à une surface corporelle de l'utilisateur et une seconde surface exposée (S2) qui fait face à la surface corporelle ; et
une pluralité d'éléments conducteurs (16, 36) s'étendant chacun le long de la direction d'accouplement et retenus par le boîtier (12) dans un état de passage à travers le boîtier (12) dans la direction d'accouplement allant de la première surface exposée (S1) à la seconde surface exposée (S2),
dans lequel chacun des éléments conducteurs (16, 36) comporte une partie contact (16A, 37) ayant une conductivité électrique et une partie électrode (16B, 38) ayant une conductivité électrique, la partie contact (16A, 37) faisant saillie à partir de la première surface exposée (S1) du boîtier (12) et étant configurée pour être connectée à un contre-contact du contre-connecteur lorsque le connecteur (11, 31, 41, 51, 61) pour l'acquisition de données biologiques est accouplé sur le contre-connecteur, la partie électrode (16B, 38) faisant saillie à partir de la seconde surface exposée (S2) du boîtier (12) et possédant une surface d'électrode (ES) configurée pour être mise en contact avec la surface corporelle de l'utilisateur et pour acquérir des données biologiques de l'utilisateur,
dans lequel l'une des deux options I et II suivantes s'applique :
option I :
chacun de la pluralité d'éléments conducteurs (16) est constitué d'un seul composant dans lequel la partie contact (16A) et la partie électrode (16B) sont formées pour être solidaires l'une de l'autre le long de la direction d'accouplement, et
la partie contact (16A) et la partie électrode (16B) ont toutes deux une forme colonnaire qui s'étend le long de la direction d'accouplement,
dans lequel chacun de la pluralité d'éléments conducteurs (16) possède une partie bride (16C) qui est en surplomb dans une direction perpendiculaire à la direction d'accouplement à partir d'une partie bordure entre la partie contact (16A) et la partie électrode (16B),
dans lequel le boîtier (12) est composé d'un isolateur supérieur (13) possédant une partie en retrait (13A) configurée pour accueillir le contre-connecteur, la première surface exposée (S1) du boîtier (12) étant formée par la surface inférieure de la partie en retrait (13A),
et un isolateur inférieur (14, 44, 64) ayant la seconde surface exposée (S2),
l'isolateur supérieur (13) comporte une pluralité de trous traversants de contact (13B) formés dans la partie en retrait (13A), chacun desquels étant configuré pour recevoir la partie contact (16A) passant à travers celui-ci et a une taille inférieure à celle de la partie bride (16C),
l'isolateur inférieur (14, 44, 64) comporte une pluralité de trous traversants d'électrode (14B) chacun desquels étant configuré pour recevoir la partie électrode (16B) passant à travers celui-ci et a une taille inférieure à celle de la partie bride (16C), et
l'isolateur supérieur (13) et l'isolateur inférieur (14, 44, 64) sont configurés pour être fixés ensemble dans un état où les parties contact (16A) de la pluralité d'éléments conducteurs (16) passent à travers la pluralité de trous traversants de contact (13B), les parties électrode (16B) de la pluralité d'éléments conducteurs (16) passent à travers la pluralité de trous traversants d'électrode (14B), et les parties bride (16C) de la pluralité d'éléments conducteurs (16) sont prises en sandwich entre l'isolateur supérieur (13) et l'isolateur inférieur (14) ;
option II :
chacun de la pluralité d'éléments conducteurs (36) est composé d'un élément de contact (37) qui forme la partie contact et d'un élément d'électrode (38) qui est configuré pour être joint à l'élément de contact (37) et forme la partie électrode,
l'élément de contact (37) possède une partie tubulaire (37A) s'étendant le long de la direction d'accouplement et pourvue dans son intérieur d'une partie en retrait (37C), et
une partie de l'élément d'électrode (38) est configurée pour être insérée dans la partie en retrait (37C) et pour entrer en contact avec une surface intérieure de la partie en retrait (37C), moyennant quoi l'élément d'électrode (38) est configuré pour être relié électriquement à l'élément de contact (37),
dans lequel l'élément de contact (37) comporte une bride côté contact (37B) en surplomb dans une direction perpendiculaire à la direction d'accouplement à partir d'une extrémité de la partie tubulaire (37A),
l'élément d'électrode (38) comporte une première partie colonnaire ronde (38A) qui s'étend le long de la direction d'accouplement et est configurée pour être insérée dans la partie en retrait (37C) de l'élément de contact (37) en tant que partie de l'élément d'électrode (38), une seconde partie colonnaire ronde (38B) qui s'étend le long de la direction d'accouplement dans une direction opposée à la première partie colonnaire ronde (38A), et une bride côté électrode (38C) en surplomb dans une direction perpendiculaire à la direction d'accouplement à partir d'une partie bordure entre la première partie colonnaire ronde (38A) et la seconde partie colonnaire ronde (38B), et
la bride côté contact (37B) et la bride côté électrode (38C) sont configurées pour être empilées dans la direction d'accouplement pour former une partie bride (36C),
dans lequel le boîtier (12) est composé d'un isolateur supérieur (13) comportant une partie en retrait (13A) configurée pour accueillir le contre-connecteur, la première surface exposée (S1) du boîtier (12) étant formée par la surface inférieure de la partie en retrait (13A), et d'un isolateur inférieur (14) possédant la seconde surface exposée (S2),
l'isolateur supérieur (13) comporte une pluralité de trous traversants de contact (13B) formés dans la partie en retrait (13A), chacun desquels étant configuré pour recevoir la partie tubulaire (37A) de l'élément de contact (37) passant à travers celui-ci et a une taille inférieure à celle de la bride côté contact (37B), l'isolateur inférieur (14) comporte une pluralité de trous traversants d'électrode (14B) dont chacun est configuré pour recevoir la seconde partie colonnaire ronde (38B) de l'élément d'électrode (38) passant à travers celui-ci et a une taille qui est inférieure à celle de la bride côté électrode (38C), et
l'isolateur supérieur (13) et l'isolateur inférieur (14) sont configurés pour être fixés ensemble dans un état où les parties tubulaires (37A) des éléments de contact (37) de la pluralité d'éléments conducteurs (16) passes à travers la pluralité de trous traversants de contact (13B),
les secondes parties colonnaires rondes (38B) des éléments d'électrode (38) de la pluralité d'éléments conducteurs (16) passent à travers la pluralité de trous traversants d'électrode (14B), et les parties bride (36C) de la pluralité d'éléments conducteurs (16) sont prises en sandwich entre l'isolateur supérieur (13) et l'isolateur inférieur (14).

2. Connecteur pour l'acquisition de données biologiques selon la revendication 1,
dans lequel l'isolateur supérieur (13) et l'isolateur inférieur (14) sont configurés pour être fixés ensemble avec l'objet de montage (21, 71) pris en sandwich entre l'isolateur supérieur (13) et l'isolateur inférieur (14), moyennant quoi le boîtier (12) est attaché à l'objet de montage (21, 71).

3. Connecteur pour l'acquisition de données biologiques selon l'une quelconque des revendications 1 et 2, comprenant une feuille de renforcement (15) qui est retenue par le boîtier (12) et qui possède une partie de fixation qui est en surplomb vers l'extérieur à partir d'une partie périphérique extérieure du boîtier,
dans lequel la partie de fixation est fixée à l'objet de montage, moyennant quoi le boîtier est attaché à l'objet de montage.

4. Connecteur pour l'acquisition de données biologiques selon la revendication 3,
dans lequel le boîtier (12) et la feuille de renforcement (15) sont formés d'une seule pièce à partir de résine isolante.

5. Connecteur pour l'acquisition de données biologiques selon l'une quelconque des revendications 1 à 4,
dans lequel le boîtier (12) comporte une partie de retenue de lubrifiant (44A) constituée d'une rainure formée au niveau de la seconde surface exposée (S2) et disposée autour de la partie électrode faisant saillie à partir de la seconde surface exposée.

6. Connecteur pour l'acquisition de données biologiques selon l'une quelconque des revendications 1 à 5, comprenant une couche adhésive sensible à la pression (52) disposée sur la seconde surface exposée (S2).

7. Connecteur pour l'acquisition de données biologiques selon la revendication 1,
dans lequel l'isolateur inférieur (14) est constitué d'un élastomère.

8. Connecteur pour l'acquisition de données biologiques selon la revendication 4,
dans lequel la résine isolante formant d'une seule pièce le boîtier (12) et la feuille de renforcement (15) est constituée d'un élastomère.

9. Connecteur pour l'acquisition de données biologiques selon l'une quelconque des revendications 1 à 8,
dans lequel la partie électrode comporte une surface d'électrode (ES) qui est exposée à partir de la seconde surface exposée (S2) et rugosifiée.

10. Connecteur pour l'acquisition de données biologiques selon l'une quelconque des revendications 1 à 9, configuré pour être monté sur un vêtement qui est l'objet de montage.
